# EUROPEAN PATENT APPLICATION

(11) **EP 4 696 333 A1**
(43) Date of publication of application: **18.02.2026**
(21) Application number: 25195635.5
(22) Date of filing: 13.08.2025
(51) Int. Cl.: A61K 49/00, C07K 14/435, G06N 3/00, G06N 3/02, G06N 3/06, C07K 14/705, G01N 33/542, G01N 33/68

(54) **MODIFICATION OF VOLTAGE-GATED CHANNELS IN NEURONS WITH FLUORESCENT DONOR-ACCEPTOR PAIRS**

(30) Priority: 13.08.2024 US 202463682707 P; 08.08.2025 US 202519294804
(71) Applicant: CCLabs Pty Ltd, Melbourne, Victoria 3000 (AU)
(72) Inventor: Kagan, Brett Joseph, Melbourne, Victoria, 3000 (AU); Doensen, Finn, Melbourne, Victoria, 3000 (AU); Watmuff, Brad, Melbourne, Victoria, 3000 (AU)
(74) Representative: Grünecker Patent- und Rechtsanwälte PartG mbB

(57) **Abstract**

Systems and techniques for producing genetically engineered ion channels (ICs) with bioluminescence resonant energy transfer (BRET) complexes and using such ion channels for efficient readout of neural activity and outputs of networks of biological neurons are described. In one embodiment, the disclosed techniques include identifying a target location in an IC, to host a target protein including a donor tag protein and an acceptor tag protein and modifying a genome of the neuron cell in a part associated with the target location in the IC. The techniques further include causing the neuron cell to express the target protein into the IC according to the modified genome. In a first (second) state of the IC, the donor tag protein is at a first (second) distance from the acceptor tag protein associated with absence (presence) of energy transfer between the donor tag protein and the acceptor tag protein.

## Description

### TECHNICAL FIELD

Embodiments of the present disclosure relate, in general, to detection and mapping of neural activity.

### BACKGROUND

Biological computing utilizes living biological matter to perform computations that can potentially be free from the limitations inherent in semiconductor technology. Biological neurons are small and have much better scalability and orders-of-magnitude higher energy efficiency than silicon-based transistor processors. Additionally, biological neural networks are fault-tolerant and in many instances can withstand the destruction of half of the biological neural network while still maintaining the network's functionality. Biological neural networks have a high neuroplasticity, which enables development of a highly adaptable intelligence suitable for many different applications.

### BRIEF DESCRIPTION OF THE DRAWINGS

The embodiments described herein will be understood more fully from the detailed description given below and from the accompanying drawings, which, however, should not be taken to limit the application to the specific embodiments, but are for explanation and understanding only.
FIG. 1 illustrates an example system architecture for a biological computing platform that can deploy bioluminescence resonant energy transfer (BRET) of proteins genetically engineered into an ion channel for readout of outputs of networks of biological neurons, according to at least one embodiment.
FIGs. 2A-2B illustrate integration of BRET complexes into an ion channel for efficient readout of neural activity and outputs of networks of biological neurons, according to at least one embodiment.
FIGs. 3A-3C illustrate operations of a BRET complex formed in an ion channel for efficient readout of neural activity and outputs of networks of biological neurons, according to at least one embodiment.
FIG. 4 is a flow diagram illustrating example methods of producing genetically engineered ion channels with BRET complexes and using such ion channels for efficient readout of neural activity and outputs of networks of biological neurons, according to at least one embodiment.

### DETAILED DESCRIPTION OF THE PRESENT DISCLOSURE

Networks for biological computing platforms are often grown in a petri dish, the neuron network being in contact with a multi-electrode array (MEA). MEAs are extensively used for recording electrical activity from neural cells and networks. These devices are integral in neuroscience research for understanding neural communication and brain function.

The MEA can be used to stimulate activity (action potential) of the neurons with electrical signals and to detect such activity. Additionally, neuron activity can be stimulated and/or detected using optical signals, e.g., delivered to and/or from the network using optical fibers. For example, data can be input into a network of neurons by sending suitably prepared electrical signals via the MEA to stimulate spontaneous electrical activity ("firing") of the neurons. Similarly, readout of the output of the network's computations may be performed by detecting electrical signals induced by the neurons in the MEA. Alternatively, or to reduce interference between the input electrical signals and the output electrical signals, optical readout techniques can be used. For example, a fluorescent genetically engineered voltage indicator (GEVI) protein, which changes its emission wavelength in response to the applied electric field, can be used to detect the action potential of a neuron. The GEVI neuron activity detection and readout techniques, however, have notable drawbacks. For example, GEVI fluorescence requires irradiation of the neurons by external light, which eventually (over the course of several days) damages the neurons.

Despite their widespread application, there are significant limitations that hinder the effectiveness and accuracy of MEA-based measurements, and our ability to stimulate cells. The first challenge is the migration of neurons over time. As neurons move away from the electrodes, the reproducibility of measurements is adversely affected, leading to data inconsistencies. Another limitation is that MEA electrodes are typically larger than individual neuronal cells, which compromises spatial resolution. This size discrepancy makes it difficult to record the activity of single neurons with high precision. In addition, traditional fluorescence readouts of activity are popular methods of measuring neuronal function but can negate the long-term recording advantages offered by MEAs by introducing phototoxicity. The light used in these systems can damage neural cells, affecting their normal function and viability over extended periods. Cross-talk is another significant issue, where the field effect across multiple electrodes can lead to erroneous readings. When multiple electrodes detect changes in the electrical field caused by the polarization of action potentials (APs) simultaneously, it can be challenging to discern the source of the signal. This issue is exacerbated in high-density MEAs, which otherwise improve spatial resolution. Cross-talk can also be a problem in neighboring or offset AC circuits, leading to further complications in signal accuracy. Finally, when multiple spikes occur at the same electrode, sorting them by the similarity of waveforms (clustering) becomes complex and highly dependent on the sampling rate. The process of spike sorting is computationally intensive, especially given the typical recording rates of 40 kHz, resulting in 50-100 data points per spike.

Aspects and embodiments of the present disclosure address these and other challenges of the existing neuron activity detection and readout technology by providing for systems and techniques that use bioluminescence resonance energy transfer (BRET) without reliance on external irradiation of neurons. The proposed systems and techniques overcome the aforementioned challenges and limitations using BRET complexes that introduce an optical control for MEA-based measurements. One major benefit of this approach involves the passive optical monitoring of neural cells and networks, which eliminates the need for light input that could artificially excite or activate tagged channels. The use of passive optical methods such as the disclosed BRET complexes ensures no artificial excitation or activation of the cells, thus preserving their natural activity and improving the accuracy and reliability of long-term recordings.

In some embodiments, a BRET complex that includes a target protein in combination with a donor tag protein (also, for brevity, referred to as a donor tag or, simply, donor herein) and an acceptor tag protein (acceptor tag or acceptor) can be genetically engineered and added to a sodium channel protein chain, e.g., by changing the genome of the neuron in such a way that gene expression of the sodium channel replaces proteins in target positions with the BRET complex. The donor tag can include one or more luciferase molecules and the acceptor tag can include one or more fluorophore molecules. The sodium channel protein chain responds to the change of electric potential across the neuron membrane during the neuron's activation-inactivation cycle by changing the shape of the sodium channel. This results in a change of distance between the donor tag and the acceptor tag. As the distance between the donor tag and the acceptor tag changes, e.g., decreases, resonant energy transfer can occur causing the fluorophore acceptor to emit light, which can be detected by readout optical detectors. Donor-acceptor pairs placed in positions that move closer together during the activation phase (e.g., when the membrane potential rises above - 55 mV and causes an activation gate of the channel to open) can be used to sense activation of the neurons. Similarly, donor-acceptor pairs placed in positions that move sufficiently close together during the inactivation phase (e.g., when the membrane potential reaches +30 mV and an inactivation gate closes) can be used to sense inactivation of the neurons. In some embodiments, luciferase (or nanoluciferase, etc.) molecule(s) can emit light in the presence of a particular substrate, e.g., coelenterazine (or furimazine for nanoluciferase). The luciferase donor oxidizes the substrate, which donates one or more electrons to the luciferase donor molecules causing the energy of the luciferase donor to increase. This additional energy is then transferred to the fluorophore acceptor and emitted as light. Since the substrate (e.g., furimazine or coelenterazine) is consumed in the oxidation process, a new substrate can be added to the petri dish while the luminescence (irradiation-free emission of light) imaging/readout is ongoing.

The advantages of the disclosed techniques include (but are not limited to) readout and/or visualization of neural activity that does not involve subjecting the neurons to light (bleaching). This facilitates sustainable and prolonged use of neural networks for biological computations as well as for observation and experimentation involving in vitro neurons. Additionally, the disclosed techniques may ensure faster detection/readout times than the existing GEVI technology. Furthermore, as networks of neurons (naturally occurring or grown in a petri dish) can include different neurons with different types of ion channels (e.g., sodium Na+ channels, potassium K+ channels, calcium Ca+ channels, etc.) or different isoforms of the same-type channels (e.g., sodium NaV1.1 and NaV1.2 channels, etc.), different BRET complexes can be used with different ion channels/isoforms, each sensed independently using different BRET complexes.

Some embodiments are discussed with reference to use of neurons having an engineered BRET complex that includes a target protein in combination with a donor tag protein and an acceptor tag protein within a neuron cell on MEAs. However, it should be understood that such modified neurons having such an engineered BRET complex may also be used for other purposes, such as to study neural activity in living beings. For example, neurons of an animal may be modified to include the engineered BRET complex, and the neural activity of such an animal may be studied by monitoring luminescence of neurons.

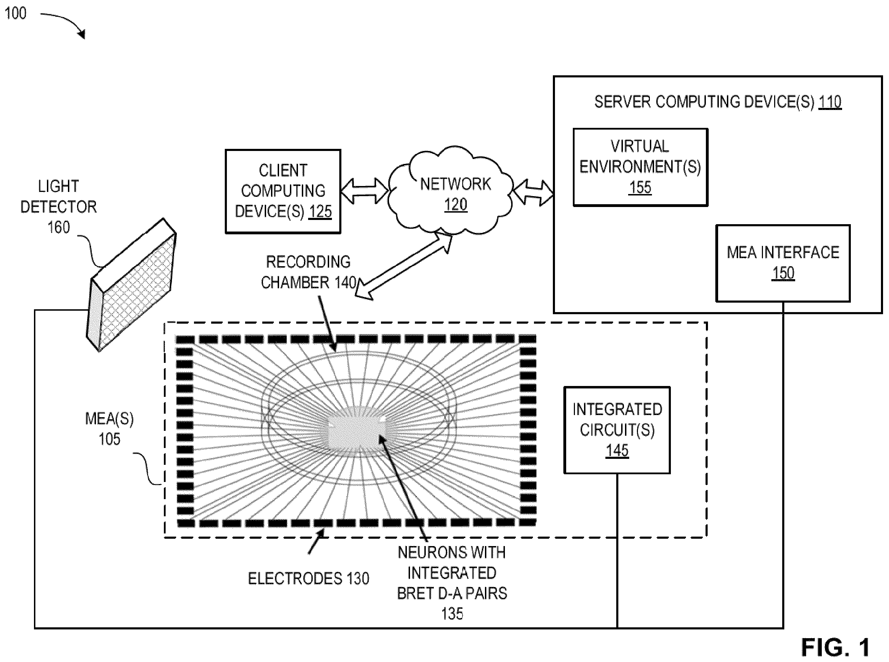

FIG. 1 illustrates an example system architecture for a biological computing platform 100 that can deploy bioluminescence resonant energy transfer (BRET) of proteins genetically engineered into an ion channel for readout of outputs of networks of biological neurons, according to at least one embodiment. It should be understood that FIG. 1 illustrates one possible use of the disclosed techniques and that various other uses are possible, including (but not limited to) providing instrumentation and techniques for research related to the structure, functionality, engineering, and/or the like of individual neurons and networks of neurons.

Biological computing platform 100 can be used to perform in vitro training of biological neurons to implement a real-time synthetic biological intelligence (SBI), among other things. Biological computing platform 100 may be a biological computing cloud platform that provides network access to biological neural networks (e.g., exposes biological neural network resources through the cloud). In one embodiment, biological computing platform 100 externalizes networks of biological neurons (e.g., cortical neurons) and provides an interface between the biological neural network and a virtual environment executed on a computing device. Accordingly, biological computing platform 100 creates an efferent (e.g., vision or other input)/efferent (e.g., motor or other output) loop between the biological neural network and the virtual environment.

As shown in FIG. 1, the biological computing platform 100 can include one or more MEA 105 connected to one or more server computing devices 110 and/or client computing device(s) 125 via a network 120. The network 120 may be a local area network, a wide area network, a private network (e.g., an intranet), a public network (e.g., the Internet), or a combination thereof. The connection between the MEA(s) 105 and the server computing device(s) 110 may include wired connections, wireless connections, or a combination thereof. Alternatively, the MEA(s) 105 may be directly connected to server computing devices(s) 110 (e.g., via a wired or wireless connection).

The server computing device(s) 110 and/or client computing device(s) 125 may include physical machines and/or virtual machines hosted by physical machines. The physical machines may be rackmount servers, desktop computers, or other computing devices. In one embodiment, the server computing device(s) 110 may include virtual machines managed and provided by a cloud provider system. Each virtual machine offered by a cloud service provider may be hosted on a physical machine configured as part of a cloud. Such physical machines are often located in a data center. The cloud provider system and cloud may be provided as an infrastructure as a service (IaaS) layer. One example of such a cloud is Amazon's^{®} Elastic Compute Cloud (EC2^{®}).

The server computing device(s) 110 may host an MEA interface 150 and one or more virtual environments 155. The MEA interface 150 and virtual environment(s) 155 may be hosted on the same server computing device 110, or may be hosted on separate server computing devices, which may be connected via the network 120.

An MEA 105 (also known as a microelectrode array) is a device that contains multiple plates or shanks through which neural signals are obtained and/or delivered. In embodiments, HD-MEA are used. The plates or shanks are generally arranged in a grid or other array, and serve as neural interfaces that connect neurons 135 to electronic circuitry. The MEA 105 includes a recording chamber 140 that houses many biological neurons 135 and/or a solution or other medium (e.g., a saline solution). These biological neurons 135 may be cultured neurons (e.g., cultured from stem cells) and/or extracted neurons (e.g., extracted from a mouse or rat brain). The biological neurons 135 may be from a generic cell line or may be from a cell line with specific traits to be tested. For example, the biological neurons 135 may be cultured from stem cells of a person having a particular genotype, or from a particular person for whom a test is to be performed, or from a person having a particular pathology. In one embodiment, the neurons 135 include cortical cells from embryonic rodent sources. In one embodiment, the neurons 135 include cortical cells from human induced pluripotent stem cell (hiPSC) sources. In some embodiments of the present disclosure, neurons 135 may include BRET complexes with integrated donor-acceptor (D-A) pairs.

Neurons can be grown or harvested from numerous sources via multiple methods. Most in-depth in vitro electrophysiological investigations on neural cells have been conducted on primary neurons. This process involves disassociating cortical cells from the dissected cortices of (typically) rodent embryos. These cells are then grown in nutrient rich medium and can be maintained on the order of months. These cultures will develop complicated morphology, with numerous dendritic and axonal connections, leading to functional biological neural networks (BNNs). In some embodiments, such cultures are developed from embryos (e.g., mouse embryos). Properties of monolayers, slices or organotypic cultures can be investigated using a relevant electrophysiological method. The development of spontaneous activity from cultures has been well documented. These developmental stages have also been modelled and found to display emergent connectivity and firing rates that showcase foundational criticality.

In one embodiment, a protocol works via inhibiting the dual SMAD signaling pathway. SMADs comprise a family of structurally similar proteins that are the main signal transducers for receptors of the transforming growth factor beta (TGF-B) superfamily, which are important for regulating cell development and growth. The abbreviation refers to the homologies to the *Caenorhabditis elegans* SMA ("small" worm phenotype) and MAD family ("Mothers Against Decapentaplegic") of genes in Drosophila. SMAD inhibition has been found to drive differentiation toward the anterior neuroectodermal lineage.

In embodiments, neuron cultures (e.g., of long-term cortical neurons and/or other types of neurons) from hiPSCs and/or other sources are implemented to form comparable networks to in vivo neuronal networks within organisms or in vitro networks found in primary neuronal cell cultures, along with appropriate biomarkers showing that cells are not only neural but also more specifically cortical. Along with circumventing ethical issues with harvesting embryonic rodents, hiPSC-derived cells have been demonstrated in embodiments to survive for greater than 6 months with maintained activity and can be grown on an exponential scale, rendering the cost per cell relatively low at high volumes. This allows neuronal 'wetware' for computation to be grown and maintained in a functional way.

Historically, neural cultures that have been studied have been sparse neural cultures (e.g., with thousands of neurons) that are two-dimensional. The sparse neural networks have been spread out on a 2D grid such that they do not overlap one another. Such cell arrangements have been used because they are easier to study by enabling individual cells to be studied. However, in some embodiments much denser arrangements of neurons are used than have been used in the past. The dense arrangements of neurons (e.g., with hundreds of thousands to millions of neurons) cause the neurons to overlap one another and form a three-dimensional arrangement in which multiple neurons may be stacked vertically in addition to being arranged on a two-dimensional grid. The dense arrangement of neurons enables the neurons to form spontaneous three-dimensional (3D) structures such as neurospheres, effectively increasing the intelligence of the biological neural network that incorporates the neurons 135. In one embodiment, the dense arrangement of neurons 135 includes at least 10,000 cells per square millimeter, at least 20,000 cells per square millimeter, or at least 50,000 cells per square millimeter. The dense arrangement of neurons enables development of computational assemblies of the neurons 135 in embodiments.

Biological neurons create ion currents through their membranes when excited, causing a change in voltage between the inside and the outside of the cell. When recording, the electrodes on an MEA transduce the change in voltage from the environment carried by ions into currents carried by electrons (electronic currents). When stimulating, electrodes may transduce electronic currents into ionic currents through the MEA. This triggers the voltage-gated ion channels on the membranes of the excitable neurons, causing the neuron to depolarize and trigger an action potential.

The size and shape of a recorded signal may depend upon the nature of the medium (e.g., solution) in which the neuron or neurons are located (e.g. the medium's electrical conductivity, capacitance, and homogeneity), the nature of contact between the neurons and the electrodes (e.g. area of contact and tightness), the nature of the electrodes (e.g. its geometry, impedance, and noise), the analog signal processing (e.g. the system's gain, bandwidth, and behavior outside of cutoff frequencies), and data sampling properties (e.g. sampling rate and digital signal processing). For the recording of a single neuron that partially covers a planar electrode, the voltage at the contact pad is approximately equal to the voltage of the overlapping region of the neuron and electrode multiplied by the ratio of the surface area of the overlapping region to the area of the entire electrode. An alternative means of predicting neuron-electrode behavior is by modeling the system using a geometry-based finite element analysis in an attempt to circumvent the limitations of oversimplifying the system in a lumped circuit element diagram.

In some embodiments, blinding is used to facilitate an ability to distinguish between detection of electrical signals generated by neurons and electrical signals generated by the MEA 105 based on instructions. Blinding prevents the stimulation of electrodes caused based on instructions from the MEA interface 150 and/or virtual environment 155 from interfering with detection of electrical signals generated by neurons 135. One or more blinding schemes may be used.

In some embodiments, MEA interface 150 and/or integrated circuits 145 may know when electrodes are stimulated and/or which electrodes are stimulated. The electrical fields generated by stimulating electrodes may be much larger than the electrical fields generated by neurons 135. Accordingly, in one embodiment, detected electrical signals are applied to a filter, which may filter out electrical fields/signals that are greater than a threshold size (e.g., that are detected by more than a threshold number of electrodes), where these electrical fields/signals are caused by active stimulation of electrodes by the integrated circuit 145 and/or MEA interface 150. Such filtering may be performed, for example, by integrated circuit 145 and/or server computing device 110. However, smaller electrical fields caused by neurons 135 may only be detected by a small number of electrodes, and may thus not be filtered out. Additionally, signals may be filtered out based on voltage. For example, electrical signals caused by electrodes 130 may have much larger voltages than electrical signals generated by neurons 135. For example, electrical signals generated by electrodes 130 may have voltages on the order of a thousandth of a volt, and electrical signals generated by neurons 135 may have voltages on the order of a millionth of a volt. Thus, electrical signals may additionally or alternatively be filtered based on voltage.

In one embodiment, a rough timing of when electrical signals are output to electrodes and/or optical signals are output via optical components is known. Knowledge of timing may not be perfect because of unpredictable delays in command delivery. Accordingly, blinding may be performed by ignoring electrical and/or optical signals output at or around the time that electrical and/or optical signals are output to the electrodes 130 and/or optical components. In one embodiment, an internal counter of commands is maintained (e.g., by server computing device 110 and/or integrated circuit 145). Each time the internal counter increments, this may indicate that new electrical and/or optical signals are output to one or more electrodes. Accordingly, in one embodiment when the internal counter increments, electrical and/or optical signals are ignored for a set amount of time.

In some embodiments, multiple blinding techniques may be combined.

In one embodiment, a blinding method (e.g., consensus blind) based on blinding all signals when >15 simultaneous large (>75 mV) spikes were detected, is implemented to block stimulation delivered by the system from being registered as cellular activity. In some embodiments, a new blinding method is implemented, which is termed 'command count blinding'. This method blinds a readout of all motor activity when a command was sent to generate any form of stimulation. During testing this was found to be significantly more robust than the previously used consensus blinding and enabled increased density and variability of sensory stimulation.

The MEA(s) 105 can be used to perform electrophysiological experiments on dissociated cell cultures (e.g., cultures of biological neurons). With dissociated neuronal cultures, the neurons spontaneously form biological neural networks. This phenomenon may be increased by using very dense neural cultures, as set forth above. The MEA(s) 105 may include an array of electrodes 130 and the recording chamber 140 that contains a living culture of biological neurons 135 in a nutrient rich solution that will keep the biological neurons alive. The array of electrodes 130 may be a planar array (e.g., a two-dimensional (2D) grid) or a three-dimensional (3D) array (e.g., a 3D matrix). The array of electrodes 130 that may be used to take measurements at 2D coordinates (or 3D coordinates) at high spatial and temporal resolution at excellent signal quality. Additionally, the array of electrodes 130 may be used to apply electrical impulses at the 2D coordinates or 3D coordinates.

Along with recording changes in electrical activity brought about from action potentials, the MEA 105 has the potential to stimulate cells at a range of voltages. Providing external electrical stimulation is relatively non-invasive to cells, and effectively elicits action potentials or responses in a comparable manner to internal electrical stimulation. With an appropriate coding scheme, external electrical stimulations are able to convey a range of information. Different coding schemes are discussed in greater detail below. Through this method there is the capacity to not only 'read' information from a neural culture, but to 'write' data into one.

One or more of the MEA(s) 105 may be an active MEA that includes an integrated circuit 145 (or multiple integrated circuits), such as a CMOS circuit. The integrated circuit(s) 145 may include processing logic (e.g., a general purpose or special purpose processor), a network adapter, a digital to analog converter (DAC), an analog to digital converter (ADC), and/or other components. The network adapter may be a wired network adapter (e.g., an Ethernet network adapter) or a wireless network adapter (e.g., a Wi-Fi network adapter), and may enable the MEA(s) 105 to connect to network 120. In one embodiment, the integrated circuit 145 includes a processing device, which may be a general purpose processor, a microcontroller, a digital signal processor (DSP), a programmable logic controller (PLC), a microprocessor or programmable logic device such as a field programmable gate array (FPGA) or a complex programmable logic device (CPLD). In one embodiment, the integrated circuit 145 includes a memory, which may be a non-volatile memory (e.g., RAM) and/or a volatile memory (e.g., ROM, Flash, etc.). In one embodiment, the integrated circuit 145 is a system on a chip (SoC) that includes the processing device, memory, network adapter, DAC, and/or ADC.

In one embodiment, one or more of the MEA(s) 105 is a passive MEA that is connected to one or more integrated circuits 145 via one or more leads and/or a printed circuit board (PCB).

In one embodiment, one or more of the MEAs 105 further includes an optical source that is capable of providing optical impulses to specified 2D coordinates in the 2D grid. The optical source may include light emitting elements (e.g., light emitting diodes (LEDs), light bulbs, lasers, etc.) that are capable of emitting light having one or more specified wavelengths. Accordingly, optogenetics may be used to manipulate neural activity. Additionally, lasers of specific wavelengths may be used for highly accurate targeting of specific neurons. The response to optical stimulation may then be measured by the electrodes in the MEA(s) 105. Unlike electrical stimulation, light stimulation manipulates specific cells (e.g., neurons) that may express a targeted opsin protein, thereby making it possible to investigate the role of a subpopulation of neurons in a neural circuit. In some embodiments, immunofluorescence of specifically modified calcium that get cleaved and activated when they enter the neurons can also be paired with a camera to image activation of neurons.

In some embodiments, neurons 135 can be stimulated electrically via electrodes 130 of MEA(s) 105 while neural activity of neurons 135 can be detected optically, e.g., using a light detector 160. For example, light detector 160 can detect light emitted by fluorophore molecules of donor-acceptor (D-A) pairs inserted (e.g., using genetic engineering) into neurons 135. Light detector 160 can be (or include) complementary metal-oxide-semiconductor (CMOS) image sensors, charge-coupled devices (CCDs), hybrid CMOS-CCD image sensors, photomultiplier tubes (e.g., an array of photocathode-based pixels), photodiodes, phototransistors, 2D imaging cameras, or any other suitable light detectors. Light detector 160 can detect visible light, infrared (IR) light, ultraviolet (UV) light, and/or waves of any other part of the electromagnetic spectrum. Delivery of light emitted by neurons 135 to the light detector 160 can be performed over air, via one or more optical fibers, waveguides, etc., or using any combination thereof. The range of light detected by light detector 160 can correspond to (or include) light emitted by the D-A pairs engineered into neurons 135. Optical signals detected by light detector 160 can be transformed into electrical signals that can be delivered to one or more integrated circuit(s) 145, MEA interface 150 of server computing device(s) 110, and/or other computing device, for any suitable processing, which can include signal filtering, de-noising, decoding, re-formatting to a suitable format that can be understood by software and/or a human operator. Mechanisms for optically detecting neural activity are discussed in greater detail below.

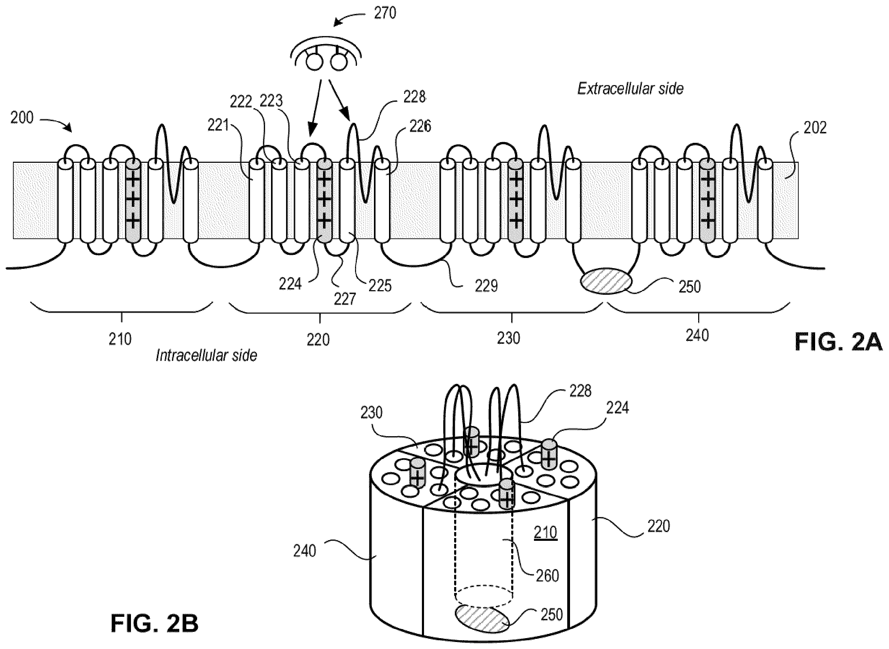

FIGs. 2A-2B illustrate integration of BRET complexes into an ion channel for efficient readout of neural activity and outputs of networks of biological neurons, according to at least one embodiment. The term "ion channel," as used throughout this disclosure, refers to a sodium (Na+) channel, potassium (K+) channel, and/or any other transmembrane channel that can transfer ions. FIG. 2A shows a planar topology of a protein chain 200 that forms an ion channel (IC) of a neuron cell. For the sake of specificity, FIG. 2A shows a sodium channel formed by the protein chain 200, but similar techniques of BRET integration can be used with other ICs. Protein chain 200 is embedded into a cell membrane 202. IC-forming protein chain 200 includes four domains 210, 220, 230, and 240. Each of the 210-240 domains can contain six membrane-spanning segments, e.g., segments 221-226 enumerated for domain 220. The fourth segment 224 has positive amino acids that, when stimulated by a transmembrane potential difference (also referred to as simply voltage herein), moves toward the outer side (top side, in FIG. 2A) of the cell membrane 202 to open the IC to the flow of ions (e.g., Na+ ions, K+ ions, etc.) into the neuron cell. Different segments are connected by short loops 227. The fourth and the fifth segments of each unit are connected by longer P-loops, e.g., P-loop 228 of domain 220. Different domains are connected by inter-domain loops 229. An inactivation gate 250 is located between the third domain 230 and the fourth domain 240.

FIG. 2B shows the protein chain 200 of FIG. 2A wrapped to form an ion channel for voltage-controlled transmembrane transport of ions. Domains 210-240 are wrapped around a central pore 260, which serves as the ion conduit. The P-loops (e.g., P-loop 228, etc.) line the inner surface of the pore 260 and serve as the activation gate of the IC. Before an action potential occurs, the IC remains in a deactivated state with the pore 260 closed off to the ions and the cell membrane 202 at a resting voltage, e.g., about -70 mV in some neurons (the sign indicating that the intracellular side of the cell membrane 202 has a lower potential than the extracellular side). At the onset of the action potential, the membrane voltage begins to rise causing the fourth segment in each of the domains (e.g., segment 224 in domain 220) to move upwards. As the membrane voltage reaches about -55 mV, the P-loops (e.g., P-loop 228 in domain 220), which serve as the activation gate, open the pore 260 to the flow of the ions from the extracellular side to the intracellular side of the neuron. The flow of the ions results in an increase of the membrane voltage to about +30 mV, at which point the inactivation gate 250 closes bringing the IC to an inactive state and starting the refractory period during which the membrane voltage gradually decreases (e.g., as a result of the outward flow of potassium ions K+ through a separate potassium channel) back to the resting potential corresponding to the falling phase of the action potential.

Referring again to FIG. 2A, to monitor various phases of the action potential, a BRET complex 270 that includes a donor-acceptor (D-A) pair can be added to the protein chain 200 at a location where the geometry of the chain changes in response to the changing membrane potential. In some embodiments, the BRET complex 270 may be added to a P-loop 228. In some embodiments, the BRET complex 270 may be added to one of the short loops 227, inter-domain loops 229 connecting different domains of the protein chain 200, portions of the protein chain that support the inactivation gate 250, and/or some other portion of the protein chain 200.

Insertion of the BRET complex 270 into ion channels can be performed using various genetic engineering techniques. In some embodiments, the techniques can include the clustered regularly interspaced palindromic repeats (CRISPR) system that uses a guide RNA and a Cas9 enzyme. The guide RNA is designed to find and bind to a specific sequence in a cell DNA. The guide RNA and the synthetic sequence may be added via the introduction of plasmid DNA, e.g., using lipofection or electroporation. The guide RNA has RNA bases that are complementary to those of the target DNA sequence in the genome, ensuring that the guide RNA only binds to the target sequence. The Cas9 enzyme binds to the guide RNA and makes a cut across both strands of the DNA at the target sequence. The cell then detects the damage to the DNA and tries to repair the damage, at which point the target mutations are introduced.

Another method of inserting the BRET complex into the host cells is via a viral vector. In such embodiments, the plasmid containing the BRET complex sequence is presented alongside viral capsule and/or reverse transcriptase sequences (themselves inserted in plasmids) and introduced to a packaging cell line, which subsequently releases viral particles that can then be purified and later introduced to the neural cell culture delivering the BRET complex. The virus produced depends on the packaging sequences chosen, and may include but are not limited to third generation lentiviruses (LVs), or replication-deficient adeno-associated viruses (AAVs).

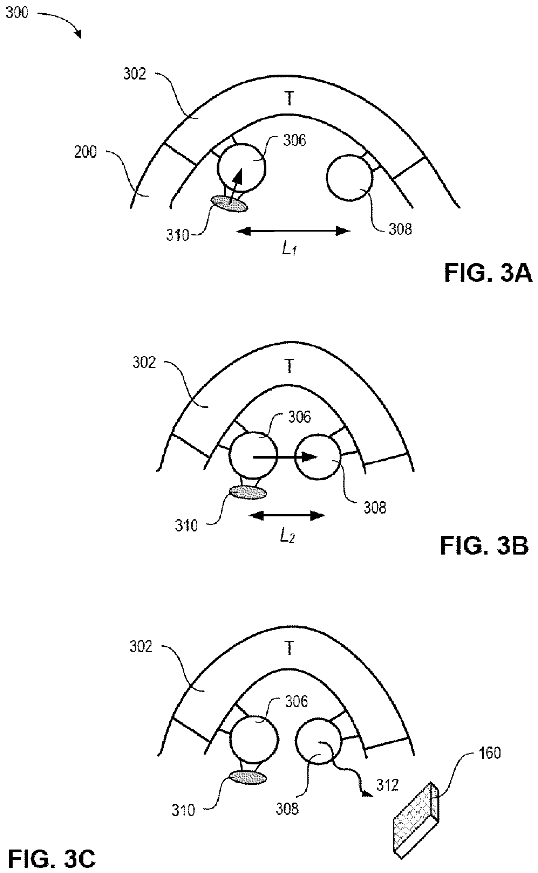

FIGs. 3A-3C illustrate operations of a BRET complex formed in an ion channel for efficient readout of neural activity and outputs of networks of biological neurons, according to at least one embodiment. As illustrated in FIG. 3A, a BRET complex 300 can include a target (T) protein 302 that can be inserted into the IC protein chain 200. In some embodiments, the donor protein 302 can be inserted into a target portion of the IC protein chain 200 that changes its geometry (shape) in response to the changes in the action potential of the neuron. Such target portions can include short loops 227, P-loops 228, inter-domain loops 229, in some embodiments. In some embodiments, the target portions can include one or more segments in any one of the domains of the IC (e.g., segments 221-226 in domain 220).

In some embodiments, the modification of the IC protein chain 200 can be genetically engineered with the target protein 302 replacing one or more proteins of the IC protein chain 200. For example, the genome of the neuron can be modified to ensure that the gene expression of the neuron results in the IC protein chain 200 having the target protein 302 in a target position within the chain. The target position(s) can be identified using the techniques that will be disclosed in more detail below. The target protein 302 can be expressed to have tags attached. More specifically, the target protein 302 can be tagged with a donor tag protein 306 capable of collecting energy, e.g., chemical energy of a valence electron, from a substrate molecule 310, as indicated schematically with a solid arrow in FIG. 3A. Additionally, the target protein 302 can be tagged with an acceptor tag protein 308, which can be a fluorophore molecule capable of emitting light when induced into an excited state (e.g., by the donor tag protein 306). In a first (dark) state of the BRET complex 300, as illustrated in FIG. 3A, the donor tag protein 306 and the acceptor tag protein 308 can be located at a distance *L*₁ that exceeds the distance of effective energy transfer. In some embodiments, *L*₁ > 10 nm and consequently, the donor tag protein 306 cannot donate energy to the acceptor tag protein 308. In some embodiments, distance *L*₁ may be different from 10 nm, e.g., be shorter than 10 nm or longer than 10 nm, and may depend on specific characteristics of the donor/acceptor, as well as their relative orientations.

In some embodiments, the donor tag protein 306 may include luciferase molecules, nanoluciferase molecules, and/or the like. The substrate 310 can include coelenterazine, furimazine, and/or similar molecules. The acceptor tag protein 308 can include green fluorescent proteins (GFP), yellow fluorescent proteins (YFP), Venus proteins, and/or the like.

As illustrated in FIG. 3B, changes in the electric potential of the cell membrane can change the shape of the IC protein chain and bring the donor tag 306 and the acceptor tag 308 closer together. Correspondingly, in a second (bright) state of the BRET complex 300, the donor tag 306 and the acceptor tag 308 can be located at a distance *L*₂ that is below the distance of effective energy transfer between the molecules. In some embodiments, *L*₂ < 10 nm. As a result, the donor tag 306 can donate energy to the acceptor tag 308, e.g., via resonant non-radiative (Forster-type) energy transfer, as illustrated schematically with the arrow in FIG. 3B. As illustrated in FIG. 3C, the excited acceptor tag 308 radiates light 312 that can be detected by light detector 160 and indicate that a particular phase of the IC activity is occurring.

The type/phase of the activity detected using the BRET complex 300 depends on the placement of the BRET complex 300 within the IC protein chain 200. In particular, placement of the BRET complex 300 at target positions with the distance decreasing (*L*₁ → *L*₂) upon activation of the IC (e.g., on the inner side of a loop that increases its curvature, as illustrated in FIGs. 3A-3C) creates optical indicators of the activation phase of the IC. Similarly, placement of the BRET complex 300 at target positions with the distance decreasing upon inactivation of the IC creates optical indicators of the inactivation phase of the IC. In some embodiments, selection of the target positions for the BRET complex 300 can be performed to minimize potential disruption of the function of the IC protein chain.

In some embodiments, the BRET complex 300 can be inserted into sodium voltage-gated channels NaV which are naturally expressed in, and vital to the function of, mature (human) neurons. For example, the sodium voltage-gated channels may include NaV1.1 isoforms associated with the function of inhibitory neurons, NaV1.2 isoforms expressed in excitatory neurons, and/or the like. Both these channels are abundantly expressed, and required for physiological function, in specific mature neurons. The NaV1.1 isoform is transcribed and translated from the human gene SCN1A, and NaV1.2 isoform is transcribed and translated from the human gene SCN2A.

Identification of target portions of the sodium (and other ion) channels for insertion of the BRET complexes can be informed by the existing x-ray crystallography studies of the NaV isoforms and studies directed to conformational changes that occur during activation and inactivation of these channels. To ensure that BRET complexes are inserted into less functionally important regions of the sodium channels and avoid disruption of the normal function of the channels, the target regions for the BRET complexes insertion may be selected among the portions of the protein that are identified in x-ray crystallography data as unstructured regions. Even though such unstructured regions are not always functionally unimportant, insertion of BRET complexes into such regions is less disruptive than modifying more structured regions. On the other hand, positioning, motion, and deformation (or other conformational changes) of the unstructured regions can be expected to correlate to conformational changes in functionally important adjacent structured regions and efficiently capture physiochemical properties of these structured regions.

In one non-limiting example, target regions of the sodium channels may include approximately 10-20 amino acid residues. The R-group of each amino acid residue within these sequences has a broad range of physiochemical properties and includes subregions in which the following residues may dominate: nonpolar/hydrophobic residues, polar/hydrophilic residues, positively charged residues, and/or negatively charged residues. In some embodiments, the target regions of the channels can be selected in conjunction with specific tags chosen for attachment to the donor and acceptor proteins, e.g., based on specific physiochemical profiles of the fluorophores, to allow for selection of fluorophore(s) (e.g., GFP, YFP, Venus protein, etc.).

In some embodiments, BRET complexes can have amino acid sequences (linkers) attached to the D-A pair, on either side of the pair. The presence of linkers may allow for a physiochemical adaptation of the inserted BRET D complexes to the target region of the ion channel, provide additional flexibility to the BRET complexes, and/or optimize the physical distance maintained between the donor tag protein and the acceptor tag protein to ensure that BRET operations (e.g., energy transfer from luciferase to the fluorophore, and emission of light by the fluorophore) correlates with the ion channel activity.

Table 1 lists some example sodium channels and donor/acceptor tags that can be used in BRET pairs for efficient readout of neural activity and outputs of networks of biological neurons (PDF stands for Protein Data Bank, OMIM stands for Online Mendelian Inheritance in Man).

| | Proteins | Genes | PDB reference ID | OMIM reference |
|---|---|---|---|---|
| Ion Channels | NaV1.1 | SCN1A | 7DTD | 182389 |
| | NaV1.1 | SCN1A | 6J8E | 182390 |
| Donor Tag | Nanoluciferase | | 7MJB; 5IBO | |
| Acceptor Tags | YFP | | 4HE4 | |
| | GFP | | 6AA6 | |
| | Venus | | 3AKO | |

In some embodiments, to identify the target sites in the ion channels, the AlphaFold Protein Structure Database (or a similar database) can be used to predict the structure of specific ion channels of interest (e.g., NaV1.1, NaV1.2, and/or the like), with and without the BRET complex inserted, including both structured and unstructured regions. A suitable visualization and analysis system, e.g., PyMOL molecular visualization tool, can be used to evaluate the degree to which the structure of the channels' functional regions changes by the insertion of the BRET complexes. Based on the information provided by visualization and analysis system, selection of the target region(s) can then be performed by balancing the following factors: (1) minimization of disruption of the normal function of the ion channel by the BRET complexes with (2) ensuring that detectable luminescence patterns correlate with one or more states of the ion channel, e.g., open state, closed state, inactive state, and/or the like. In some embodiments, a specific BRET complex may be capable of detecting just one of these states, e.g., an open state or a closed state. In some embodiments, a specific BRET complex can be used to detect multiple such states.

Some examples target sites for NaV1.1 and NaV1.2 sodium channels have been identified as follows:
1. SCN1A F1529_N1530insYFP Q1563 _K1564insnLuc;
2. SCN1A V804_N805insnLuc;
3. SCN1A S383 _D384insVenus T1424_G1425insnLuc;
4. SCN1A F1529 _N1530insYFP T2022_A2023insnLuc2022;
5. SCN1A S383 _D384insVenus N1528 _F1529insnLuc;
6. SCN1A S383_D384insVenus N1790_P1791insnLuc;
7. SCN2A C768_P769insGFP M856_D857insLuc;
8. SCN2A C768_P769insGFP S2014 _P2015insnLuc.

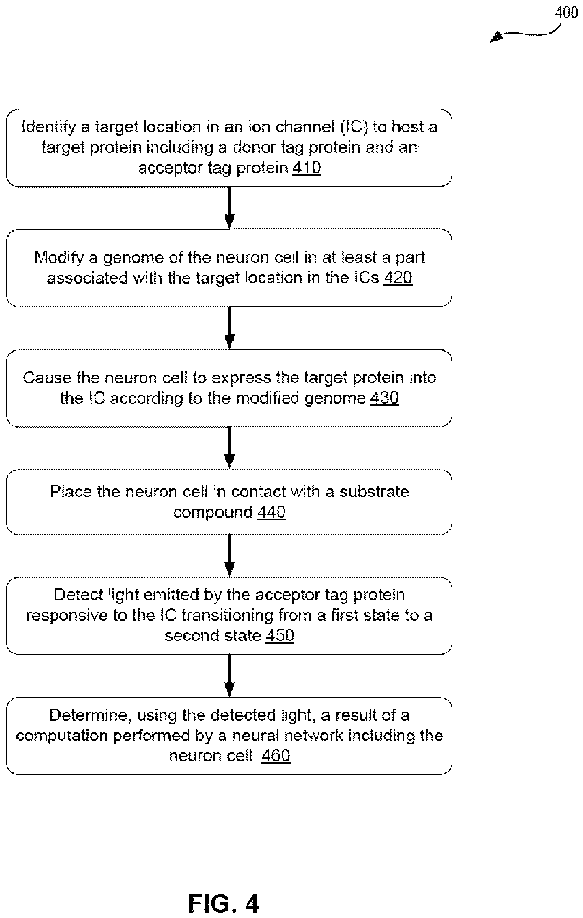

FIG. 4 is a flow diagram illustrating an example method 400 of producing genetically engineered ion channels with BRET complexes and using such ion channels for efficient readout of neural activity and outputs of networks of biological neurons, according to at least one embodiment. For simplicity of explanation, method 400 is depicted and described as a series of acts. However, acts in accordance with this disclosure can occur in various orders and/or concurrently and with other acts not presented and described herein. Furthermore, not all illustrated acts may be performed to implement the methods in accordance with the disclosed subject matter. For example, in some embodiments, operations of blocks 410-430 associated with producing neuron cells with genetically modified ion channels can be performed independently of operations of blocks 440-460 associated with detecting electrical activity of neuron cells using genetically modified ion channels.

At block 410, method 400 can include identifying a target location in the ion channel (IC), the target location to host a target protein (mutant protein) that includes a donor tag protein and an acceptor tag protein. In some embodiments, the IC may include a sodium channel, a potassium channel, a calcium channel, and/or the like. In some embodiments, operations of block 410 can include identifying one or more unstructured regions in an x-ray image of the IC. Operations of block 410 can further include selecting, within the one or more unstructured regions, the target location in view of at least an amount of disruption of a function of the IC caused by a replacement of a native protein of the IC with the target protein.

At block 420, method 400 may include modifying a genome of the neuron cell in at least a part associated with the target location in the IC. At block 430, method 400 can continue with causing the neuron cell to express the target protein into the IC according to the modified genome. In some embodiments, the target protein can include the donor tag protein and the acceptor tag protein. In some embodiments, the donor tag protein can include a luciferase protein, and the acceptor tag protein can include the GFP, the YFP, the Venus protein, and/or the like.

In some embodiments, in a first state of the IC, the donor tag protein is at a first distance from the acceptor tag protein. The first distance can be associated with absence of a non-radiative energy transfer (NRET) between the donor tag protein and the acceptor tag protein. In a second state of the IC, the donor tag protein is at a second distance from the acceptor tag protein. The second distance can be associated with the presence of the NRET between the donor tag protein and the acceptor tag protein. For example, the first distance can be above 10 nm and the second distance can be below 10 nm.

In some embodiments, the first state of the IC is associated with activation of the IC (or inactivation of the IC) and the second state of the IC is associated with inactivation of the IC (or activation of the IC). In some embodiments, the first state of the IC is associated with an open IC (closed IC) and the second state of the IC is associated with the closed IC (open IC). In some embodiments, a transition from the first state of the IC to the second state of the IC can be responsive to a conformational change of the IC. The conformational change of the IC can be associated with a change of electric potential of a membrane of the neuron cell.

In some embodiments, in the second state of the IC, the acceptor tag protein emits light responsive to the NRET from the donor tag protein. In some embodiments, an energy transferred in the NRET can be generated in an oxidation reaction between the donor tag protein and a substrate molecule.

Once the neuron cell having the above described engineered properties has been grown, the neuron cell may be used for one or more purposes.

In one embodiment, at block 440, method 400 can include placing the neuron cell in contact with a substrate compound. In some embodiments, substrate compound can include furimazine, coelenterazine, and/or the like. In one embodiment, at block 450, method 400 can include detecting a light emitted by the acceptor tag protein responsive to the IC transitioning from the first state to the second state. In some embodiments, the IC transitioning from the first state to the second state can be caused by a natural (e.g., spontaneous) electrical activity of the neuron cell or an external electrical stimulation of the neuron cell.

In one embodiment, at block 460, method 400 can include determining, using the detected light, a result of a computation performed by a neural network comprising the neuron cell.

Some portions of the detailed description have been presented in terms of algorithms and symbolic representations of operations on data bits within a computer memory. These algorithmic descriptions and representations are the means used by those skilled in the data processing arts to most effectively convey the substance of their work to others skilled in the art. An algorithm is here, and generally, conceived to be a self-consistent sequence of steps leading to a desired result. The steps are those requiring physical manipulations of physical quantities. Usually, though not necessarily, these quantities take the form of electrical or magnetic signals capable of being stored, transferred, combined, compared, and otherwise manipulated. It has proven convenient at times, principally for reasons of common usage, to refer to these signals as bits, values, elements, symbols, characters, terms, numbers, or the like.

It should be borne in mind, however, that all of these and similar terms are to be associated with the appropriate physical quantities and are merely convenient labels applied to these quantities. Unless specifically stated otherwise, as apparent from the following discussion, it is appreciated that throughout the description, discussions utilizing terms such as "receiving", "converting", "sending", or the like, may refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

Embodiments of the present disclosure also relate to an apparatus for performing the operations herein. This apparatus may be specially constructed for the discussed purposes, and/or it may comprise a general purpose computer system selectively programmed by a computer program stored in the computer system. Such a computer program may be stored in a computer readable storage medium, such as, but not limited to, any type of disk including floppy disks, optical disks, CD-ROMs, and magnetic-optical disks, read-only memories (ROMs), random access memories (RAMs), erasable programmable read only memories (EPROMs), electrically erasable programmable read only memories (EEPROMs), magnetic disk storage media, optical storage media, flash memory devices, other type of machine-accessible storage media, or any type of media suitable for storing electronic instructions, each coupled to a computer system bus.

It is to be understood that the above description is intended to be illustrative, and not restrictive. Many other embodiments will be apparent to those of skill in the art upon reading and understanding the above description. Although the present disclosure has been described with reference to specific example embodiments, it will be recognized that the disclosure is not limited to the embodiments described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

## Claims

1. A method of genetically modifying an ion channel of a neuron cell, comprising:
identifying a target location in the ion channel (IC), the target location for a target protein pair comprising a donor tag protein and an acceptor tag protein; and
modifying a genome of the neuron cell such that the neuron cell expresses a modified IC comprising the target protein pair at the target location,
wherein, when expressed by the neuron cell, the target protein pair exhibits a first state or a second state,
wherein in the first state, the donor tag protein is at a first distance from the acceptor tag protein, the first distance associated with absence of a non-radiative energy transfer (NRET) between the donor tag protein and the acceptor tag protein, and
wherein in the second state, the donor tag protein is at a second distance from the acceptor tag protein, the second distance associated with presence of the NRET between the donor tag protein and the acceptor tag protein.

2. The method of claim 1, wherein the donor tag protein comprises a luciferase protein.

3. The method of claim 1, wherein the acceptor tag protein comprises at least one of:
a green fluorescent protein (GFP),
a yellow fluorescent protein (YFP), or
a Venus protein.

4. The method of claim 1, wherein the IC comprises at least one of:
a sodium channel,
a potassium channel, or
a calcium channel.

5. The method of claim 1, wherein the first distance is above 10 nm and the second distance is below 10 nm.

6. The method of claim 1, wherein the first state of the IC is associated with at least one of activation of the IC or inactivation of the IC, and wherein the second state of the IC is associated with another one of activation of the IC or inactivation of the IC.

7. The method of claim 1, wherein the first state of the IC is associated with at least one of an open IC or a closed IC, and wherein the second state of the IC is associated with another one the open IC or the closed IC.

8. The method of claim 1, wherein a transition from the first state of the IC to the second state of the IC is responsive to a conformational change of the IC.

9. The method of claim 1, wherein in the second state of the IC the acceptor tag protein emits light responsive to the NRET from the donor tag protein.

10. The method of claim 1, wherein an energy transferred in the NRET is generated in an oxidation reaction between the donor tag protein and a substrate molecule.

11. The method of claim 1, wherein identifying the target location in the IC comprises:
identifying one or more unstructured regions in an x-ray image of the IC; and
selecting, within the one or more unstructured regions, the target location in view of at least an amount of disruption of a function of the IC caused by a replacement of a native protein of the IC with the target protein.

12. A system comprising:
a neural network comprising a neuron cell, wherein the neuron cell comprises an ion channel (IC) genetically modified by a mutant protein that comprises a donor tag protein and an acceptor tag protein; and
an optical detector to detect a light emitted by the acceptor tag protein responsive to the IC transitioning from a first state to a second state,
wherein in the first state, the donor tag protein is at a first distance from the acceptor tag protein, the first distance associated with absence of a non-radiative energy transfer (NRET) between the donor tag protein and the acceptor tag protein, and
wherein in the second state, the donor tag protein is at a second distance from the acceptor tag protein, the second distance associated with presence of the NRET between the donor tag protein and the acceptor tag protein.

13. The system of claim 12, wherein the donor tag protein comprises a luciferase protein, and wherein the acceptor tag protein comprises at least one of:
a green fluorescent protein (GFP),
a yellow fluorescent protein (YFP), or
a Venus protein.

14. The system of claim 12, wherein the first state of the IC is associated with at least one of activation of the IC or inactivation of the IC, and wherein the second state of the IC is associated with another one of activation of the IC or inactivation of the IC.

15. The system of claim 12, further comprising:
a processing device to determine, using the detected light, a result of a computation performed by the neural network.
